# EUROPEAN PATENT APPLICATION

(11) **EP 4 821 939 A1**
(43) Date of publication of application: **14.10.2026**
(21) Application number: 24899952.6
(22) Date of filing: 06.12.2024
(51) Int. Cl.: C07J 43/00, A61K 31/58, A61P 25/00

(54) **CRYSTAL FORM OF GABAA RECEPTOR MODULATOR, PREPARATION METHOD THEREFOR AND USE THEREOF**

(30) Priority: 08.12.2023 CN 202311688180
(71) Applicant: Shandong Luye Pharmaceutical Co., Ltd., Yantai, Shandong 264670 (CN)
(72) Inventor: LIU, Huifeng, Yantai, Shandong 264670 (CN); WANG, Guanghui, Yantai, Shandong 264670 (CN); SUN, Jiuchang, Yantai, Shandong 264670 (CN); RUAN, Zheng, Yantai, Shandong 264670 (CN)
(74) Representative: karo IP
(86) International application number: PCT/CN2024/137361
(87) International publication number: WO 2025/119319

(57) **Abstract**

The present invention relates to a crystal form of a GABAA receptor modulator, a preparation method, and the use. Specifically, provided are a crystal form of a compound of formula (I), a preparation method, and the use in the preparation of a GABAA receptor modulator drug.

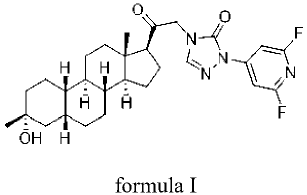

## Description

### TECHNICAL FIELD

The present disclosure belongs to the field of pharmaceutical chemistry and particularly relates to crystal forms of a GABAA receptor modulator, preparation methods therefor, and use thereof.

### BACKGROUND

GABA (γ-aminobutyric acid) is an important inhibitory neurotransmitter in the central nervous system of vertebrates. It regulates neuronal excitability and the secretion of other neurotransmitters through GABA receptors. GABA receptors have 3 subtypes: the GABA_{A} receptor, the GABA_{B} receptor, and the GABA_{C} receptor. The GABA_{A} receptor is the most abundant and important of the GABA receptors. The GABA_{A} receptor is distributed throughout the nervous system. It is a pentameric chloride ion channel receptor mainly consisting of two α subunits (α1-α6), two β subunits (β1-β3), and one additional subunit (γ1-γ3, δ, ε, π, or θ). These subunits can randomly constitute different types of GABA_{A} subtypes. The GABA_{A} subtypes expressed on the cell membrane of neurons mainly consist of five subunits (containing α, β, and γ subunits and two additional subunits). After the GABA_{A} receptor binds to GABA, the chloride ion channel on the receptor opens, selectively allowing chloride ions to flow inward into neurons. This leads to hyperpolarization of the cell membrane, thereby reducing the generation of action potentials by neurons and ultimately decreasing neuronal excitability.

The GABA_{A} receptor is associated with emotional changes such as anxiety, tension, and depression. Alcohol, barbiturates, steroids, and benzodiazepines can bind to this receptor and then modulate the activity of the GABA receptor, thereby producing a range of effects. A large amount of data confirms that the GABA_{A} receptor has a special binding site for steroid compounds. Steroids are potent broad-spectrum positive modulators of the GABA_{A} receptor. They bind to multiple sites of the GABA_{A} receptor, producing anxiolytic, anesthetic, antiepileptic, sedative, and antidepressant effects.

PCT/CN2023/099369 provides a compound with a structure as shown below. The test results show that the compound has good GABA_{A} receptor modulator activity.

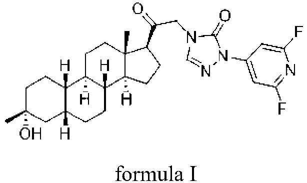

The pharmaceutical form of a compound, such as its crystal form or salt, often affects the chemical stability of the drug. Variations in crystallization and storage conditions may lead to changes in the crystal form structure of the compound and sometimes even result in the formation of other crystal forms. Generally, amorphous drug products lack a regular crystal form structure, which often leads to defects such as poor product stability, fine crystallization, filtration difficulties, proneness to agglomeration, and poor flowability. Given the importance of solid drug salt forms and crystal forms and their stability in clinical treatment, in-depth research on crystal forms of the compound of formula I is of great significance for the development of drugs that are suitable for industrial production and have good biological activity.

### SUMMARY

In one aspect, the present disclosure provides a crystal form A of the compound of formula I, having, in an X-ray powder diffraction pattern using Cu-Kα radiation, characteristic diffraction peaks at the following 2θ angles: 6.752±0.2°, 13.684±0.2°, 15.748±0.2°, and 17.744±0.2°.

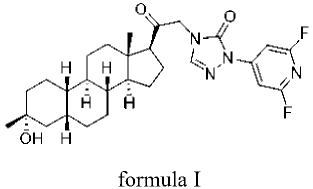

In some embodiments, the crystal form A of the compound of formula I has, in an X-ray powder diffraction pattern using Cu-Kα radiation, characteristic diffraction peaks at the following 2θ angles: 6.752±0.2°, 13.684±0.2°, 15.483±0.2°, 15.748±0.2°, and 17.744±0.2°.

In some embodiments, the crystal form A of the compound of formula I has, in an X-ray powder diffraction pattern using Cu-Kα radiation, characteristic diffraction peaks at the following 2θ angles: 6.752±0.2°, 9.943±0.2°, 12.794±0.2°, 13.684±0.2°, 14.750±0.2°, 15.483±0.2°, 15.748±0.2°, and 17.744±0.2°.

In some embodiments, the crystal form A of the compound of formula I has, in an X-ray powder diffraction pattern using Cu-Kα radiation, characteristic diffraction peaks at the following 2θ angles: 6.752±0.2°, 9.943±0.2°, 12.794±0.2°, 13.684±0.2°, 14.750±0.2°, 15.483±0.2°, 15.748±0.2°, 17.744±0.2°, 20.669±0.2°, and 22.253±0.2°.

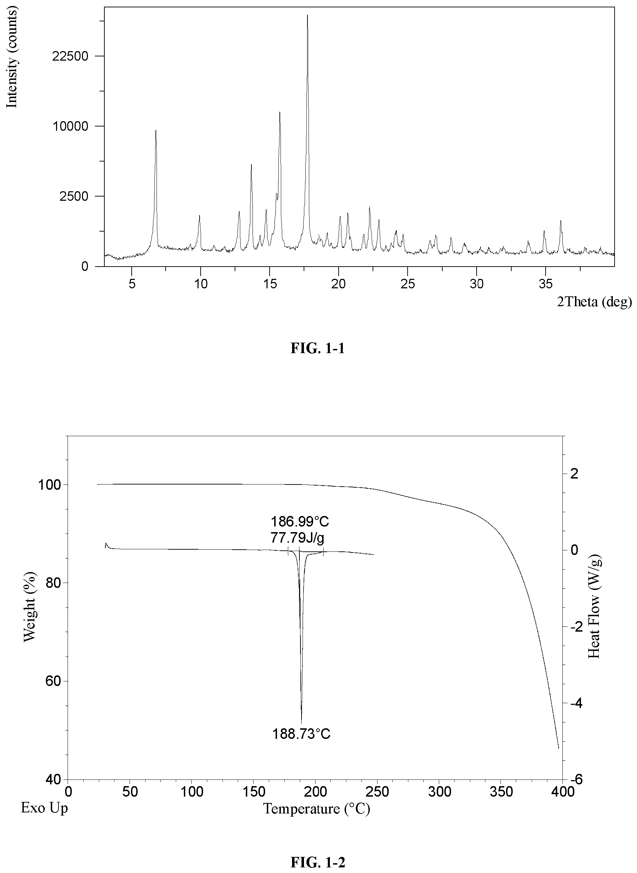

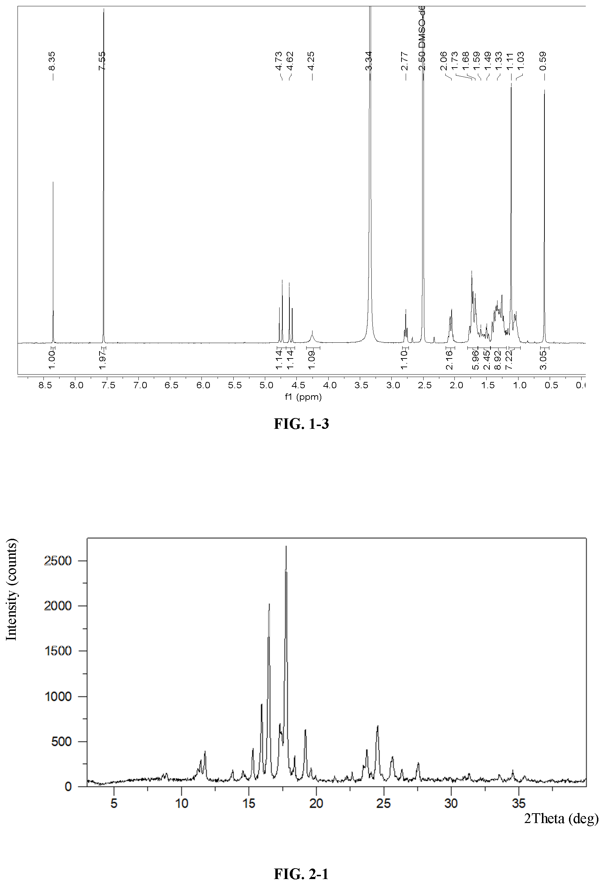

In some embodiments, the crystal form A of the compound of formula I has an X-ray powder diffraction pattern using Cu-Kα radiation as shown in FIG. 1-1.

In one aspect, the present disclosure provides a crystal form B of the compound of formula I, having, in an X-ray powder diffraction pattern using Cu-Kα radiation, characteristic diffraction peaks at the following 2θ angles: 15.962±0.2°, 16.497±0.2°, 17.275±0.2°, and 17.757±0.2°.

In some embodiments, the present disclosure provides a crystal form B of the compound of formula I, having, in an X-ray powder diffraction pattern using Cu-Kα radiation, characteristic diffraction peaks at the following 2θ angles: 11.753±0.2°, 15.312±0.2°, 15.962±0.2°, 16.497±0.2°, 17.275±0.2°, 17.757±0.2°, 18.395±0.2°, 19.191±0.2°, 23.754±0.2°, and 24.571±0.2°.

In some embodiments, the present disclosure provides a crystal form B of the compound of formula I, having, in an X-ray powder diffraction pattern using Cu-Kα radiation, characteristic diffraction peaks at the following 2θ angles: 11.428±0.2°, 11.753±0.2°, 15.312±0.2°, 15.962±0.2°, 16.497±0.2°, 17.275±0.2°, 17.757±0.2°, 19.191±0.2°, 23.754±0.2°, 24.571±0.2°, and 25.672±0.2°.

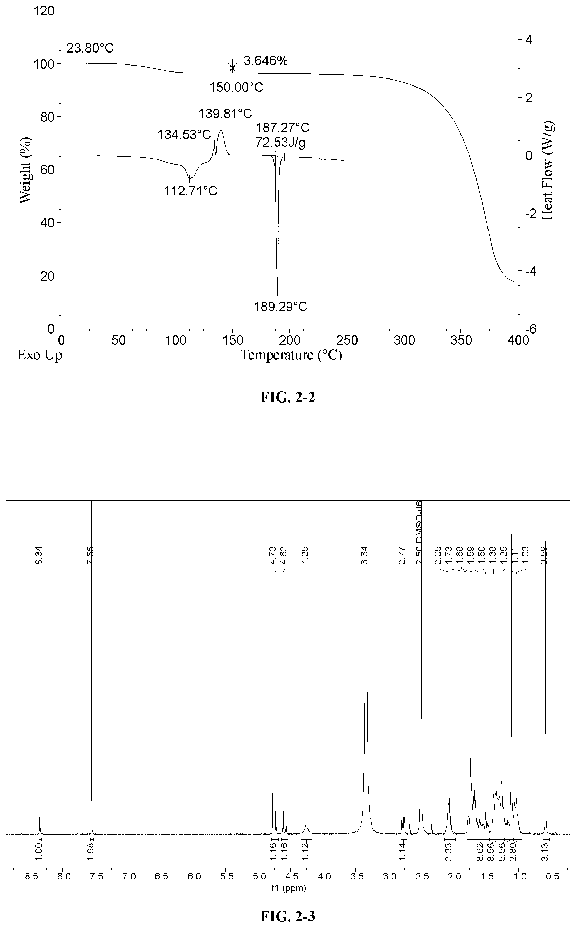

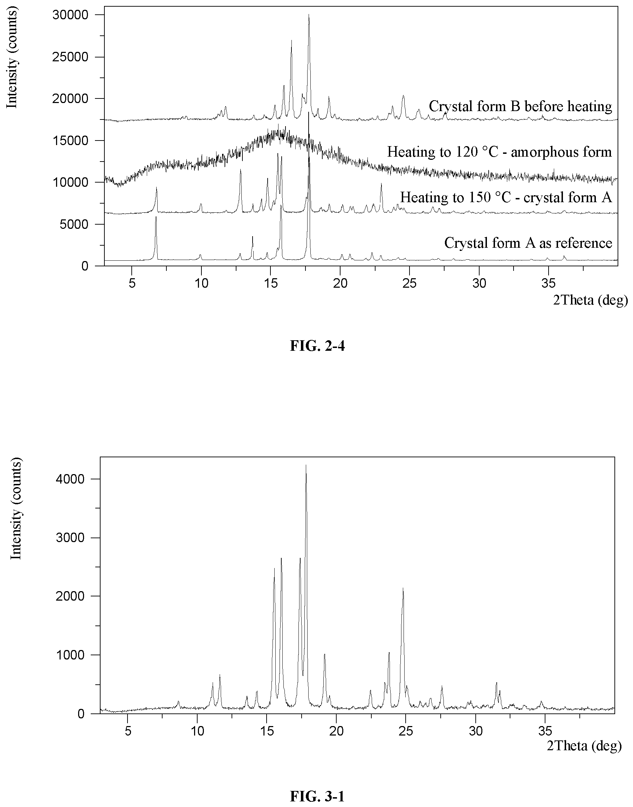

In some embodiments, the crystal form B of the compound of formula I has an X-ray powder diffraction pattern using Cu-Kα radiation as shown in FIG. 2-1.

In some embodiments, the crystal form B of the compound of formula I is a hydrate crystal form.

In one aspect, the present disclosure provides a preparation method for the crystal form B of the compound of formula I, wherein the crystal form A is subjected to a method, such as gas-liquid diffusion, stirring at room temperature, stirring at 50 °C, or cyclic heating and cooling, to give the crystal form B.

In some embodiments, the present disclosure provides a preparation method for the crystal form B of the compound of formula I, wherein the crystal form A is stirred with cyclic heating and cooling in an NPA/H₂O (1:3, v/v) solvent system to give the crystal form B.

In some embodiments, the present disclosure provides a preparation method for the crystal form B of the compound of formula I, wherein the crystal form A is stirred at room temperature in an EtOH/H₂O (855:145, v/v) system to give the crystal form B.

In some embodiments, the present disclosure provides a preparation method for the crystal form B of the compound of formula I, wherein the crystal form A is dissolved in ethyl acetate, and n-heptane is then added as an anti-solvent to give the crystal form B.

In some embodiments, the present disclosure provides a preparation method for the crystal form B of the compound of formula I, wherein the crystal form A is stirred at 50 °C in an ACN/H₂O (1:3, v/v) system to give the crystal form B.

In one aspect, the present disclosure provides a methanol solvate crystal form C of the compound of formula I, having, in an X-ray powder diffraction pattern using Cu-Kα radiation, characteristic diffraction peaks at the following 2θ angles: 15.574±0.2°, 16.065±0.2°, 17.384±0.2°, and 17.834±0.2°.

In some embodiments, the methanol solvate crystal form C of the compound of formula I has, in an X-ray powder diffraction pattern using Cu-Kα radiation, characteristic diffraction peaks at the following 2θ angles: 11.112±0.2°, 11.631±0.2°, 15.574±0.2°, 16.065±0.2°, 17.384±0.2°, 17.834±0.2°, 19.166±0.2°, 23.507±0.2°, 23.791±0.2°, and 24.810±0.2°.

In some embodiments, the methanol solvate crystal form C of the compound of formula I has, in an X-ray powder diffraction pattern using Cu-Kα radiation, characteristic diffraction peaks at the following 2θ angles: 11.112±0.2°, 11.631±0.2°, 14.307±0.2°, 15.574±0.2°, 16.065±0.2°, 17.384±0.2°, 17.834±0.2°, 19.166±0.2°, 23.507±0.2°, 23.791±0.2°, 24.810±0.2°, and 31.474±0.2°.

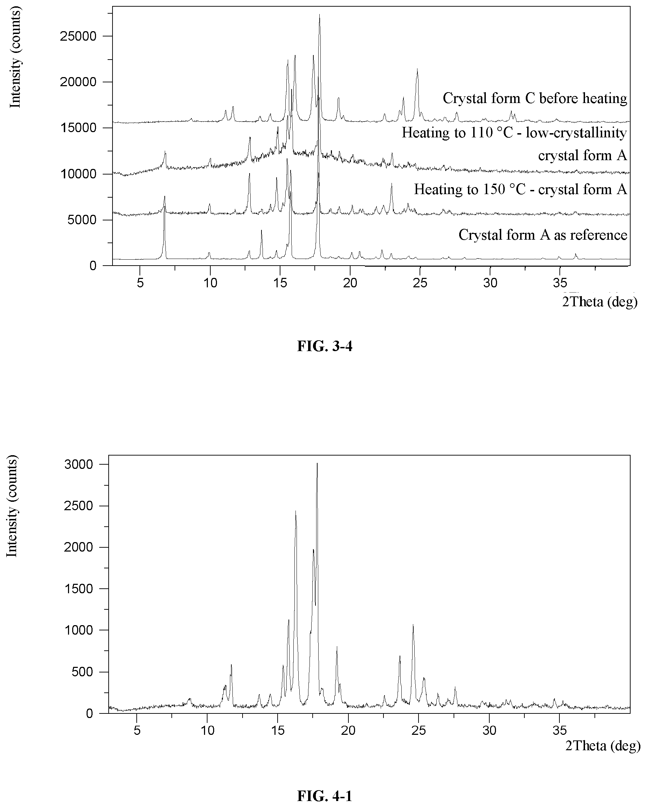

In some embodiments, the methanol solvate crystal form C of the compound of formula I has an X-ray powder diffraction pattern using Cu-Kα radiation as shown in FIG. 3-1.

In some embodiments, the methanol solvate crystal form C of the compound of formula I has a molar ratio of MeOH to the compound of formula I of about 0.8-1.2:1.

In one aspect, the present disclosure provides a preparation method for the methanol solvate crystal form C of the compound of formula I, wherein the crystal form A of the compound of formula I is stirred at room temperature in a solvent and dried at room temperature to give the crystal form C. The solvent is a MeOH/H₂O system.

In one aspect, the present disclosure provides a methanol solvate crystal form D of the compound of formula I, having, in an X-ray powder diffraction pattern using Cu-Kα radiation, characteristic diffraction peaks at the following 2θ angles: 15.782±0.2°, 16.288±0.2°, 17.575±0.2°, and 17.819±0.2°.

In some embodiments, the methanol solvate crystal form D of the compound of formula I has, in an X-ray powder diffraction pattern using Cu-Kα radiation, characteristic diffraction peaks at the following 2θ angles: 11.731±0.2°, 15.398±0.2°, 15.782±0.2°, 16.288±0.2°, 17.314±0.2°, 17.575±0.2°, 17.819±0.2°, and 19.192±0.2°.

In some embodiments, the methanol solvate crystal form D of the compound of formula I has, in an X-ray powder diffraction pattern using Cu-Kα radiation, characteristic diffraction peaks at the following 2θ angles: 11.731±0.2°, 15.398±0.2°, 15.782±0.2°, 16.288±0.2°, 17.314±0.2°, 17.575±0.2°, 17.819±0.2°, 19.192±0.2°, 23.653±0.2°, and 24.595±0.2°.

In some embodiments, the methanol solvate crystal form D of the compound of formula I has, in an X-ray powder diffraction pattern using Cu-Kα radiation, characteristic diffraction peaks at the following 2θ angles: 11.256±0.2°, 11.731±0.2°, 15.398±0.2°, 15.782±0.2°, 16.288±0.2°, 17.314±0.2°, 17.575±0.2°, 17.819±0.2°, 19.192±0.2°, 19.414±0.2°, 23.653±0.2°, 24.595±0.2°, and 25.43±0.2°.

In some embodiments, the methanol solvate crystal form D of the compound of formula I has an X-ray powder diffraction pattern using Cu-Kα radiation as shown in FIG. 4-1.

In some embodiments, the methanol solvate crystal form D of the compound of formula I has a molar ratio of MeOH to the compound of formula I of about 0.5.

In one aspect, the present disclosure provides a preparation method for the methanol solvate crystal form D of the compound of formula I, wherein the crystal form A of the compound of formula I is stirred at room temperature in a solvent and dried at room temperature to give the crystal form D. The solvent is a MeOH/H₂O system.

The present disclosure further provides a pharmaceutical composition comprising any one of the aforementioned crystal forms of the compound of formula I and a pharmaceutically acceptable carrier. The pharmaceutical composition may be prepared in various pharmaceutically acceptable dosage forms, such as tablets, capsules, oral liquids, granules, injections, or various sustained-release and controlled-release preparations. The pharmaceutical composition may be administered orally or parenterally (e.g., intravenously, subcutaneously, or topically). The administration dose may be appropriately adjusted based on the patient's age, sex, and disease type, and the daily dose is generally about 1-200 mg.

In one aspect, the present disclosure further provides use of any one of the aforementioned crystal forms of the compound of formula I or the pharmaceutical composition in the preparation of a GABAA receptor modulator medicament.

The present disclosure further provides use of any one of the aforementioned pharmaceutically acceptable crystal forms of the compound of formula I or the pharmaceutical composition in the preparation of a medicament for treating CNS-related diseases. The CNS-related diseases include, but are not limited to, sleep disorder (e.g., insomnia), mood disorder (e.g., depression (e.g., major depressive disorder (MDD)), manic disorder, dysthymic disorder (e.g., minor depressive disorder), bipolar disorder (e.g., type I and/or type II), anxiety disorder (e.g., generalized anxiety disorder (GAD) and social anxiety disorder), stress, post-traumatic stress disorder (PTSD), compulsive disorder (e.g., obsessive-compulsive disorder (OCD))), schizophrenia spectrum disorder (e.g., schizophrenia and schizoaffective disorder), convulsive disorder (e.g., epilepsy (e.g., status epilepticus (SE)), epileptic seizures), memory and/or cognitive disorders (e.g., attention disorders (e.g., attention deficit hyperactivity disorder (ADHD)), dementia (e.g., dementia of the Alzheimer type, dementia with Lewy bodies, and vascular dementia), movement disorder (e.g., Huntington's disease, Parkinson's disease, and essential tremor), personality disorder (e.g., antisocial personality disorder and obsessive-compulsive personality disorder), autism spectrum disorder (ASD) (e.g., autism and monogenetic causes of autism, e.g., synaptophathy), e.g., Rett syndrome, fragile X syndrome, and Angelman syndrome), pain (e.g., neuropathic pain, injury-related pain syndrome, acute pain, and chronic pain), traumatic brain injury (TBI), vascular diseases (e.g., stroke, ischemia, and vascular malformation), substance abuse disorder and/or withdrawal syndrome (e.g., addiction to opiates, cocaine, and/or alcohol), and tinnitus.

The present disclosure further provides use of any one of the aforementioned pharmaceutically acceptable crystal forms of the compound of formula I or the pharmaceutical composition in the preparation of a medicament for treating depression. The depression is selected from the group consisting of minor depression, major depressive disorder (MDD), persistent depressive disorder (PDD), psychotic depression, postpartum depression (PPD), and seasonal affective disorder.

### Definitions and Explanations

Unless otherwise specified, the following terms and phrases used herein are intended to have the following meanings. A particular term or phrase, when not specifically defined, should not be considered indefinite or unclear, but should be construed according to its common meaning. When referring to a trade name, it is intended to refer to its corresponding commercial product or its active ingredient.

The term "pharmaceutically acceptable" is described in the present disclosure in terms of compounds, compositions, and/or dosage forms that are, within the scope of sound medical judgment, suitable for contact with tissues of humans and animals without undue toxicity, irritation, allergic response, or other problems or complications, and commensurate with a reasonable benefit/risk ratio.

The compounds of the present disclosure may have asymmetric carbon atoms (optical centers) or double bonds. Racemates, diastereoisomers, geometric isomers, and individual isomers all fall within the scope of the present disclosure.

The compounds of the present disclosure may be present in particular geometric or stereoisomeric forms. All such compounds are contemplated herein, including cis and trans isomers, (-)- and (+)-enantiomers, (*R*)- and (*S*)-enantiomers, diastereoisomers, (*D*)-isomers, (*L*)-isomers, and racemic mixtures and other mixtures thereof, such as enantiomerically or diastereoisomerically enriched mixtures, all of which fall within the scope of the present disclosure. Additional asymmetric carbon atoms may be present in substituents such as alkyl. All such isomers and mixtures thereof fall within the scope of the present disclosure.

The term "pharmaceutically acceptable carrier" refers to any preparation or carrier medium that is capable of delivering an effective amount of the active substance of the present disclosure, does not interfere with the biological activity of the active substance, and has no toxic or side effects on a host or patient; representative carriers include, but are not limited to: binders, fillers, lubricants, disintegrants, wetting agents, dispersing agents, solubilizers, suspending agents, etc.

The present disclosure is intended to include all isotopes of atoms present in the compounds of the present disclosure. Isotopes include those atoms having the same atomic number but different mass numbers. As a general and nonlimiting example, isotopes of hydrogen include deuterium and tritium. Isotopes of carbon include ¹³C and ¹⁴C. Isotopically labeled compounds of the present disclosure can generally be prepared by conventional techniques known to those skilled in the art or by methods similar to those described herein using an appropriate isotopically labeled reagent in place of the non-labeled reagent otherwise used.

### BRIEF DESCRIPTION OF THE DRAWINGS

FIGs. 1-1, 1-2, and 1-3 show an XRPD pattern, TGA/DSC thermograms, and a ¹H NMR spectrum of the crystal form A of the compound of formula I, respectively.
FIGs. 2-1, 2-2, 2-3, and 2-4 show an XRPD pattern, TGA/DSC thermograms, a ¹H NMR spectrum, and a comparison of XRPD patterns before and after heating of the crystal form B of the compound of formula I, respectively.
FIGs. 3-1, 3-2, 3-3, and 3-4 show an XRPD pattern, TGA/DSC thermograms, a ¹H NMR spectrum, and a comparison of XRPD patterns before and after heating of the methanol solvate crystal form C of the compound of formula I, respectively.
FIGs. 4-1, 4-2, 4-3, and 4-4 show an XRPD pattern, TGA/DSC thermograms, a ¹H NMR spectrum, and a comparison of XRPD patterns before and after heating of the methanol solvate crystal form D of the compound of formula I, respectively.

### DETAILED DESCRIPTION

The present disclosure is further illustrated below with reference to specific examples and test examples, but these examples do not limit the scope of the present disclosure in any way.

Test conditions for the instruments used in the experiments:

### X-ray powder diffraction (XRPD)

X-ray powder diffraction data for samples were collected under ambient conditions using a Malvern Panalytical Aeris X-ray powder diffractometer. A sample (several milligrams) was taken, spread on a zero-background silicon sample tray, flattened with a glass slide with weighing paper sandwiched between the sample and the glass slide, and analyzed. The X-ray tube used a Cu target (Kα), with a Kα2/Kα1 intensity ratio of 0.50 (1.54439 Å/1.5406 Å). The X-ray emitter had a power of 300 W, a voltage of 40 kV, and a current of 7.5 mA. The test range was 3-40° (2θ), the scanning speed was 0.14°/s, and the step size was 0.02° (2θ).

### Thermogravimetric analyzer (TGA)

Thermogravimetric data for samples were collected using a TA Discovery TGA5500 thermogravimetric analyzer. A sample (several milligrams) was taken, placed in a Tzero aluminum pan (the sample was automatically weighed by the instrument in the test), and heated from room temperature to the target temperature in a N₂ atmosphere, with a N₂ flow rate of 25 mL/min and a heating rate of 10 °C/min.

### Differential scanning calorimeter (DSC)

Thermal data for samples were collected using a TA Discovery DSC2500 differential scanning calorimeter. A sample (several milligrams) was weighed into a Tzero aluminum pan, and the pan was sealed with a Tzero hermetic lid. The sample was heated to the target temperature (before the decomposition temperature) in a N₂ atmosphere, with a N₂ flow rate of 50 mL/min and a heating rate of 10 °C/min.

### Proton nuclear magnetic resonance spectroscopy (¹H NMR)

Samples were dissolved in DMSO-d₆ to form solutions with a concentration of about 2-10 mg/mL, and proton nuclear magnetic resonance spectroscopic data for the samples were collected using a Bruker AVANCE NEO 400 MHZ nuclear magnetic resonance spectrometer.

### Example 1. Synthesis and Characterization of Compound of Formula I

### 1.1. Synthesis of intermediate M2

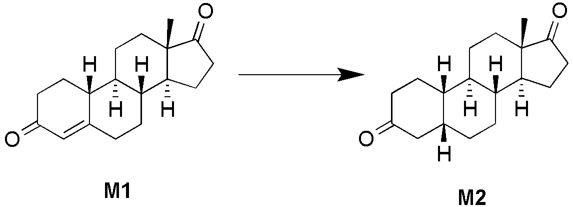

M1 (200 g, 734 mmol, 1.00 eq) and HBr (5.96 g, 35.4 mmol, 4.00 mL, 0.048 eq) were added to THF (1400 mL), and Pd/C (10.0 g, 9.43 mmol, 0.013 eq) was then added. The system was stirred at 25 °C under H₂ (1.00 MPa) for 24 h. After the system was filtered and concentrated, the solid was ground and dissolved in 600 mL of acetone. After filtration, a white crude product was obtained. The crude product was separated and purified by column chromatography (dichloromethane/methanol (10:1-5:1)) to give M2 (180 g, white solid).

### 1.2. Synthesis of intermediate M3

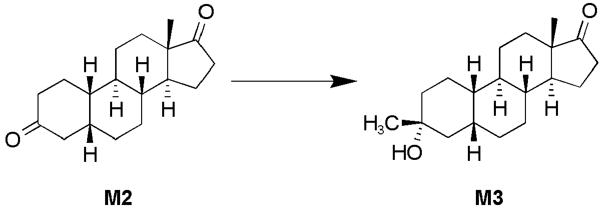

M2 (100 g, 364 mmol, 1.00 eq) was added to 1 L of toluene, and methylaluminum bis(2,6-di-tert-butyl-4-methylphenoxide) (525 g, 1.09 mol, 571 mL, 3.00 eq) was then added. The system was stirred at -60 °C for 1 h, and methylmagnesium bromide (3 M, 364 mL, 3.00 eq) was then added at -60 °C. The system was stirred at -60 °C for 3 h, and saturated NH₄Cl (500 mL) was then added. Then, the mixture was filtered, and the filter cake was washed with ethyl acetate (200 mL × 2). The organic phases were combined and then washed with saturated brine (100 mL), dried over anhydrous sodium sulfate, and filtered. The organic phase was concentrated under reduced pressure, and the residue was separated and purified by column chromatography (dichloromethane/methanol (100:1-10:1)) to give M3 (46 g, white solid, 98 g of M2 recovered).

### 1.3. Synthesis of intermediate M4

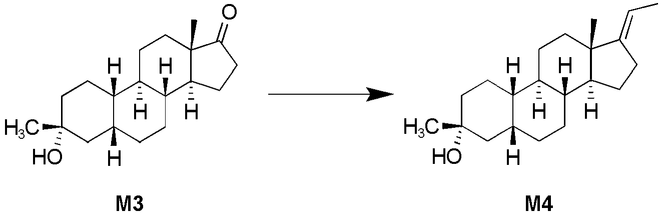

Ethyltriphenylphosphonium bromide (102 g, 275 mmol, 4.00 eq) was added to THF (120 mL) at 0 °C, and t-BuOK (30.9 g, 275 mmol, 4.00 eq) in THF (200 mL) was then added. Then, the system was stirred at 60 °C for 1 h. Then, M3 (20.0 g, 68.9 mmol, 1.00 eq) was dissolved in THF (120 mL), and the solution was then added to the system. The system was stirred at 60 °C for 13 h, quenched with an NH₄Cl solution (150 mL) under ice bath conditions, and then extracted with ethyl acetate (150 mL × 2). The organic phases were combined and then washed with saturated brine (50 mL), dried over anhydrous sodium sulfate, and filtered. The organic phase was concentrated under reduced pressure, and the residue was separated and purified by column chromatography (dichloromethane/methanol (100: 1 to 3:1)) to give M4 (18.3 g, 60.6 mmol, yield: 88.0%).

### 1.4. Synthesis of intermediate M5

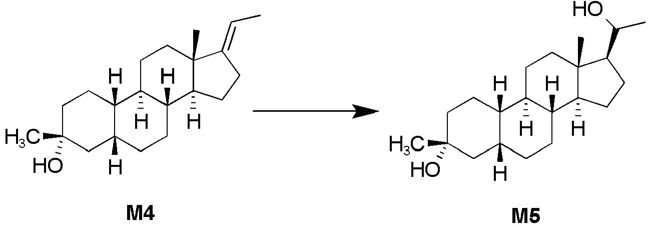

M4 (18.3 g, 60.6 mmol, 1.00 eq) was dissolved in THF (180 mL), and BH₃•Me₂S (10.0 M, 30.3 mL, 5.00 eq) was then added at 0 °C. The system was stirred at 25 °C for 3 h, and 3.00 M NaOH (100 mL) and H₂O₂ (86.5 g, 763 mmol, 73.3 mL, 12.6 eq) were then added dropwise at 0 °C. The system was left to react at 25 °C for 4 h and filtered, and the filtrate was then quenched with a Na₂SO₃ solution (60.0 mL × 2) and extracted with ethyl acetate (150 mL × 2). The organic phases were combined and then washed with saturated brine (100 mL), dried over anhydrous sodium sulfate, and filtered. The organic phase was concentrated under reduced pressure, and the residue was separated and purified by column chromatography (dichloromethane/methanol (100: 1-3: 1)) to give M5 (13.0 g, 40.6 mmol, yield: 66.9%).

### 1.5. Synthesis of intermediate M6

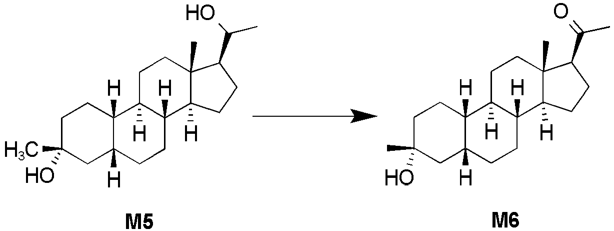

M5 (13.0 g, 40.6 mmol, 1.00 eq) was dissolved in dichloromethane (130 mL), and PCC (17.5 g, 81.1 mmol, 2.00 eq) was then added at 0 °C. The system was stirred at 25 °C for 3 h and filtered, and the filtrate was then quenched with a Na₂SO₃ solution (100 mL) and extracted with dichloromethane (100 mL × 2). The organic phases were combined and then washed with saturated brine (100 mL), dried over anhydrous sodium sulfate, and filtered. The organic phase was concentrated under reduced pressure, and the residue was separated and purified by column chromatography (dichloromethane/methanol (100:1-3:1)) to give M6 (9.03 g, 28.3 mmol, yield: 69.9%).

### 1.6. Synthesis of intermediate M7

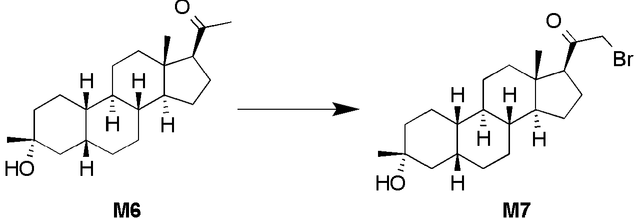

M6 (9.03 g, 28.3 mmol, 1.00 eq) was dissolved in MeOH (90.0 mL), and Br₂ (4.53 g, 28.3 mmol, 1.46 mL, 1.00 eq) and HBr (927 mg, 5.50 mmol, 622 µL, 0.190 eq) were then added at 0 °C. Then, the system was stirred at 25 °C for 12 h, quenched with a NaHCO₃ solution (50 mL), and extracted with ethyl acetate (50.0 mL × 2). The organic phases were combined and then washed with saturated brine (50 mL), dried over anhydrous sodium sulfate, and filtered. The organic phase was concentrated under reduced pressure, and the residue was separated and purified by column chromatography (dichloromethane/methanol (100:1-1:1)) to give M7 as a white solid (7.1 g, 17.8 mmol, yield: 63.0%). ¹H NMR (400 MHz, CDCl3) δ 3.99-3.85 (m, 2H), 2.92-2.69 (m, 1H), 2.25-2.12 (m, 1H), 1.98-1.58 (m, 9H), 1.53-1.28 (m, 11H), 1.27-1.18 (m, 3H), 1.15-1.03 (m, 3H), 0.64 (s, 3H).

### 1.7. Synthesis of intermediate 1-2

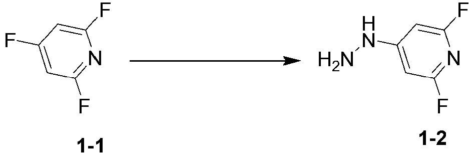

Compound 1-1 (20.0 g, 7.51 mmol, 1.00 eq) was added to a 1 L three-necked flask in a nitrogen atmosphere, and tetrahydrofuran (400 mL) was then added at 20-30 °C. The mixture was well stirred, and N₂H₄•H₂O (23.03 g, 450.89 mmol, 22.32 mL, 98% purity, 3.0 eq) was added at 20-30 °C. Then, the system was stirred at 60 °C for 16 h, and the reaction solution was directly concentrated to give compound 1-2 as a pale yellow solid (23.3 g, crude). ¹H NMR (400 MHz, DMSO-d6) δ 8.42 (br s, 1H), 6.28-5.98 (m, 2H), 4.47 (br s, 2H).

### 1.8. Synthesis of intermediate 1-3

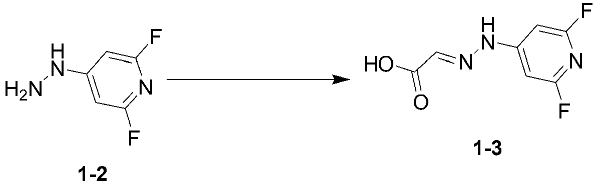

Compound 1-**2** (20 g, 137.83 mmol, 1 eq) was added to a 500 mL flask, and water (200 mL) was added. Then, the reaction solution was cooled to 0 °C, and hydrochloric acid (12 M, 22.97 mL, 2.0 eq) and glyoxylic acid (20.41 g, 137.83 mmol, 15.34 mL, 1.00 eq) were added. The reaction solution was stirred at 0 °C for 1 h and filtered, and the filter cake was concentrated to give compound 1-3 as a yellow solid (26.0 g, crude). 1H NMR (400 MHz, DMSO-d6) δ 12.44 (s, 1H), 7.39 (s, 1H), 6.65 (br s, 2H).

### 1.9. Synthesis of intermediate 1-4

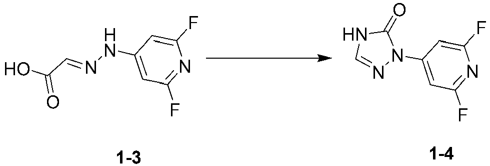

Compound 1-3 (8.00 g, 39.78 mmol, 1 eq) was added to a 250 mL three-necked flask, and toluene (100 mL), triethylamine (8.05 g, 79.55 mmol, 11.07 mL, 2.0 eq), and DPPA (10.95 g, 39.78 mmol, 8.59 mL, 1 eq) were added to the reaction system at 20-30 °C. The reaction solution was stirred at 100 °C for 16 h and then concentrated to give a brown oily crude product. The crude product was purified by preparative HPLC (column: Welch Xtimate C18 250 × 50 mm × 10 µm; mobile phase: [water (HCl)-ACN]; gradient: 0%-35% B over 25 min) to give compound 1-4 as a yellow powdery solid (1.50 g, 7.27 mmol, 36.55% yield). MS (ESI) m/z = 199.2 [M+H]+. ¹H NMR (400 MHz, DMSO-d6) δ 12.43 (br s, 1H), 8.32 (s, 1H), 7.54 (s, 2H).

### 1.10. Synthesis of compound of formula I

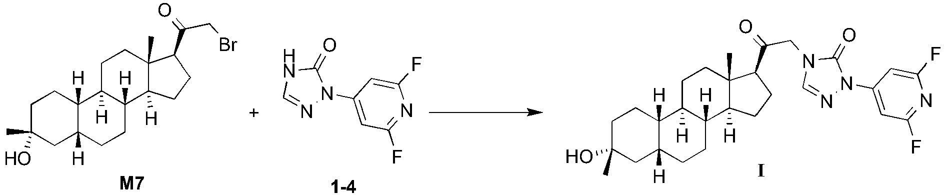

K₂CO₃ (634 mg, 4.59 mmol, 10.0 eq), compound M7 (182 mg, 459 µmol, 1.00 eq), and compound 1-4 (100 mg, 505 µmol, 1.10 eq) were added to THF (4.0 mL). Then, the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure to give a crude product, and the crude product was separated and purified by HPLC to give the compound of formula I as a pale yellow solid (153 mg, 185.2 µmol, yield: 62.3%, identified as the crystal form A of the compound of formula I). MS (ESI) m/z = 497.2[M+H]+. ¹H NMR (400 MHz, DMSO-d6) δ = 8.35 (s, 1H), 7.55 (s, 2H), 4.84-4.49 (m, 2H), 4.25 (s, 1H), 4.03 (q, J = 7.2 Hz, 1H), 2.77 (br t, J = 8.8 Hz, 1H), 2.06 (br d, J = 9.6 Hz, 2H), 1.78-0.98 (m, 23H), 0.59 (s, 3H).

### Example 2. Preparation of Crystal Form A of Compound of Formula I

1.00 g of the crystal form A of the compound of formula I was weighed out and completely dissolved in 12 mL of a solvent system of absolute ethanol by heating at 75-78 °C. The solution was slowly cooled to 20-30 °C and stirred for 2 h. The obtained compound was identified as the crystal form A of the compound of formula I (yield: 74%, purity: 99.7%). The product was characterized by XRPD, ¹H NMR, TGA, and DSC. The XRPD pattern is shown in FIG. 1-1, and the characteristic peak positions are shown in Table 1. The TGA/DSC results (FIG. 1-2) show nearly no weight loss before decomposition, and an endothermic signal at 188.73 °C (peak temperature). The ¹H NMR result (FIG. 1-3) indicates an anhydrous crystal form.

**Table 1. XRPD pattern analysis data for the crystal form A of the compound of formula I**

| No. | 2θ±0.2 (°) | Relative intensity (%) | No. | 2θ±0.2 (°) | Relative intensity (%) |
|---|---|---|---|---|---|
| 1 | 6.752 | 30.64 | 16 | 22.253 | 5.46 |
| 2 | 9.943 | 3.68 | 17 | 22.916 | 3.25 |
| 3 | 11.706 | 0.17 | 18 | 24.188 | 1.73 |
| 4 | 12.794 | 4.44 | 19 | 24.677 | 1.37 |
| 5 | 13.260 | 0.37 | 20 | 26.622 | 0.80 |
| 6 | 13.684 | 16.97 | 21 | 27.031 | 1.31 |
| 7 | 14.750 | 4.88 | 22 | 28.127 | 1.13 |
| 8 | 15.483 | 8.29 | 23 | 29.066 | 0.51 |
| 9 | 15.748 | 38.22 | 24 | 30.876 | 0.16 |
| 10 | 17.744 | 100.00 | 25 | 31.889 | 0.19 |
| 11 | 18.560 | 1.17 | 26 | 33.744 | 0.72 |
| 12 | 19.175 | 1.58 | 27 | 34.880 | 1.70 |
| 13 | 20.119 | 3.75 | 28 | 36.088 | 3.08 |
| 14 | 20.669 | 4.34 | 29 | 37.823 | 0.17 |
| 15 | 21.823 | 1.34 | 30 | 38.949 | 0.18 |

### Example 3. Preparation of Crystal Form B of Compound of Formula I

20.38 mg of the crystal form A was weighed out and stirred with cyclic heating and cooling in a n-propanol/H₂O (1:3, v/v) solvent system to give the crystal form B (purity: 99.3%).

The crystal form B was characterized by XRPD, ¹H NMR, TGA, and DSC. The XRPD pattern is shown in FIG. 2-1, and the characteristic peak positions are shown in Table 2. The TGA/DSC results (FIG. 2-2) show that the sample had a weight loss of about 3.6 wt% below 150 °C (the weight loss corresponding to one water molecule was 3.4 wt%), and had two endothermic signals at 112.71 °C and 189.29 °C (peak temperatures) and two continuous exothermic signals at 134.5 °C and 139.8 °C. The ¹H NMR spectrum (DMSO-*d₆*) is shown in FIG. 2-3. There was no significant solvent residue in the sample. The crystal form B was subjected to a heating experiment. A comparison of the XRPD patterns before and after heating is shown in FIG. 2-4. The crystal form was transformed into an amorphous form after being heated to 120 °C (after the first endothermic signal), and was transformed into the crystal form A after being heated to 150 °C. According to the characterization data and the heating experiment, it can be seen that the crystal form B was a hydrate crystal form.

Another preparation method: about 200 mg of the crystal form A of the compound of formula I was stirred at room temperature in an EtOH/H₂O (855:145, v/v) system for 3 days and dried at room temperature (26 °C, 57% RH) for about 6 h to give a crystal form (yield: 93.4%, HPLC purity: 99.5%). The crystal form obtained by this method was also identified as a hydrate crystal form B.

**Table 2. XRPD pattern analysis data for the crystal form B of the compound of formula I**

| No. | 2θ±0.2 (°) | Relative intensity (%) | No. | 2θ±0.2 (°) | Relative intensity (%) |
|---|---|---|---|---|---|
| 1 | 8.627 | 2.04 | 18 | 22.663 | 3.93 |
| 2 | 8.921 | 2.85 | 19 | 23.471 | 6.01 |
| 3 | 11.428 | 8.59 | 20 | 23.754 | 13.69 |
| 4 | 11.753 | 12.45 | 21 | 24.086 | 3.69 |
| 5 | 13.792 | 4.75 | 22 | 24.571 | 22.90 |
| 6 | 14.544 | 4.46 | 23 | 25.505 | 7.51 |
| 7 | 15.312 | 13.00 | 24 | 25.672 | 10.26 |
| 8 | 15.962 | 32.41 | 25 | 26.332 | 5.28 |
| 9 | 16.497 | 72.74 | 26 | 27.549 | 7.58 |
| 10 | 17.275 | 24.54 | 27 | 29.492 | 1.82 |
| 11 | 17.757 | 100.00 | 28 | 29.876 | 1.47 |
| 12 | 18.395 | 10.06 | 29 | 31.318 | 3.67 |
| 13 | 19.191 | 21.35 | 30 | 33.553 | 2.79 |
| 14 | 19.613 | 5.38 | 31 | 34.547 | 4.43 |
| 15 | 19.939 | 1.70 | 32 | 35.420 | 2.53 |
| 16 | 21.332 | 1.84 | 33 | 37.467 | 0.67 |
| 17 | 22.263 | 1.94 | 34 | 38.431 | 0.50 |

### Example 4. Preparation of Methanol Solvate Crystal Form C of Compound of Formula I

7.88 mg of the crystal form A and 8.90 mg of the crystal form B were weighed out, stirred at room temperature in a methanol system for about 5 days, and dried at room temperature (26 °C, 57% RH) for about 3 h to give the crystal form C.

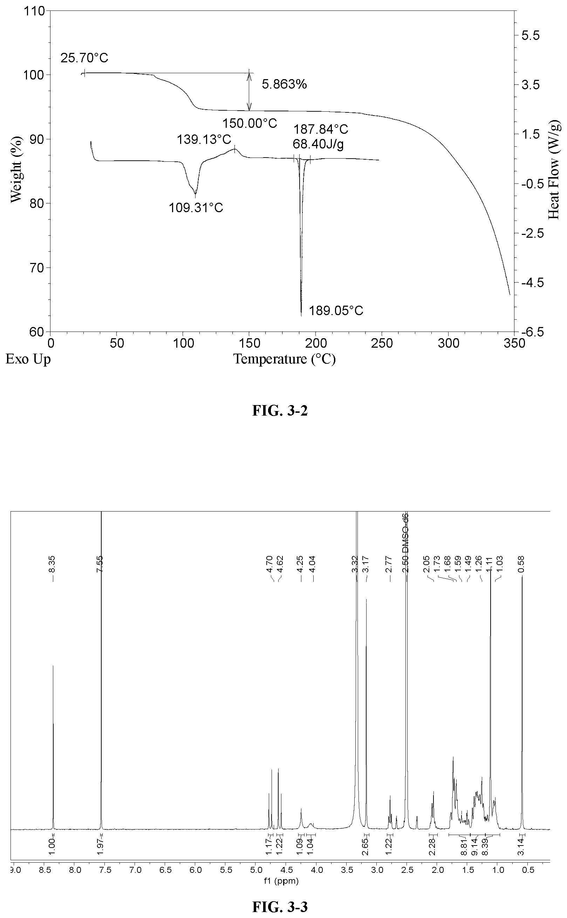

The product was characterized by XRPD, 1H NMR, TGA, and DSC. The XRPD pattern is shown in FIG. 3-1, and the characteristic peak positions are shown in Table 3. The TGA/DSC results (FIG. 3-2) show that the sample had a weight loss of about 5.9 wt% below 150 °C, and had two endothermic signals at 109.3 °C and 189.05 °C (peak temperatures) and one exothermic signal at 139.1 °C. The ¹H NMR spectrum (DMSO-*d₆*) is shown in FIG. 3-3. The molar ratio of MeOH to the compound of formula I in the sample was 1.1 (the corresponding mass fraction was 6.4%). The crystal form was subjected to a heating experiment. A comparison of the XRPD patterns before and after heating is shown in FIG. 3-4. The crystal form was transformed into a low-crystallinity crystal form A after being heated to 110 °C (after the first endothermic signal), and was transformed into the crystal form A after being heated to 150 °C. According to the characterization data and the heating experiment, it can be seen that the crystal form was a methanol solvate.

**Table 3. XRPD pattern analysis data for the methanol solvate crystal form C of the compound of formula I**

| No. | 2θ±0.2 (°) | Relative intensity (%) | No. | 2θ±0.2 (°) | Relative intensity (%) |
|---|---|---|---|---|---|
| 1 | 8.690 | 2.86 | 15 | 24.810 | 49.38 |
| 2 | 11.112 | 10.22 | 16 | 25.086 | 8.70 |
| 3 | 11.631 | 14.16 | 17 | 26.026 | 3.00 |
| 4 | 13.579 | 4.03 | 18 | 26.408 | 2.26 |
| 5 | 14.307 | 7.13 | 19 | 26.781 | 4.56 |
| 6 | 15.574 | 56.37 | 20 | 27.563 | 8.86 |
| 7 | 16.065 | 61.47 | 21 | 29.659 | 2.91 |
| 8 | 17.384 | 62.52 | 22 | 30.727 | 1.17 |
| 9 | 17.834 | 100.00 | 23 | 31.474 | 10.26 |
| 10 | 19.166 | 22.75 | 24 | 31.723 | 7.46 |
| 11 | 19.505 | 5.62 | 25 | 32.621 | 1.66 |
| 12 | 22.466 | 6.80 | 26 | 33.507 | 1.64 |
| 13 | 23.507 | 10.91 | 27 | 34.732 | 3.03 |
| 14 | 23.791 | 22.97 | - | - | - |

### Example 5. Preparation of Methanol Solvate Crystal Form D of Compound of Formula I

7.46 mg of the crystal form A and 8.12 mg of the crystal form B were weighed out, stirred at room temperature in a methanol/H₂O (840:160, v/v) system for about 5 days, and dried at room temperature (26 °C, 57% RH) for about 3 h to give the crystal form D.

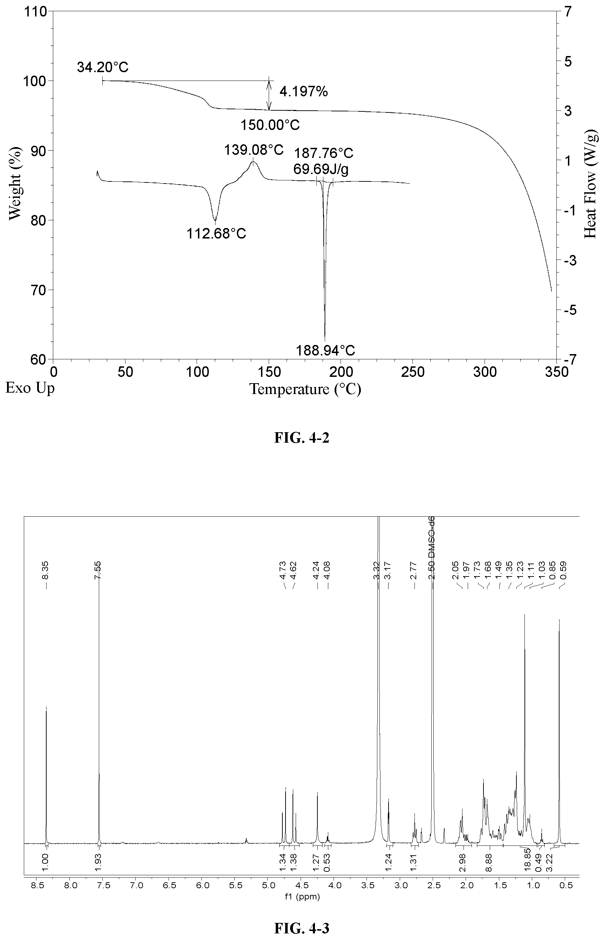

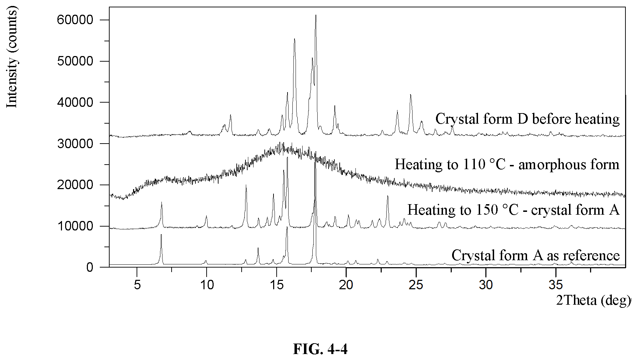

The product was characterized by XRPD, ¹H NMR, TGA, and DSC. The XRPD pattern is shown in FIG. 4-1, and the characteristic peak positions are shown in Table 4. The TGA/DSC results (FIG. 4-2) show that the sample had a weight loss of 4.2 wt% below 150 °C, and had two endothermic signals at 112.7 °C and 188.9 °C (peak temperatures) and one exothermic signal at 139.1 °C. The ¹H NMR spectrum (DMSO-*d₆*) is shown in FIG. 4-3. The molar ratio of MeOH to the API in the sample was 0.5 (the corresponding mass fraction was 3.2%). The crystal form was subjected to a heating experiment. A comparison of the XRPD patterns before and after heating is shown in FIG. 4-4. The crystal form was transformed into an amorphous form after being heated to 110 °C (after the first endothermic signal), and was transformed into the crystal form A after being heated to 150 °C. According to the characterization data and the heating experiment, it can be seen that the crystal form was a methanol solvate.

**Table 4. XRPD pattern analysis data for the methanol solvate crystal form D of the compound of formula I**

| No. | 2θ±0.2 (°) | Relative intensity (%) | No. | 2θ±0.2 (°) | Relative intensity (%) |
|---|---|---|---|---|---|
| 1 | 8.815 | 3.20 | 15 | 22.570 | 4.76 |
| 2 | 11.256 | 8.09 | 16 | 23.653 | 19.85 |
| 3 | 11.731 | 16.92 | 17 | 24.595 | 33.75 |
| 4 | 13.706 | 4.74 | 18 | 25.433 | 11.55 |
| 5 | 14.497 | 5.12 | 19 | 26.347 | 6.13 |
| 6 | 15.398 | 17.61 | 20 | 27.115 | 2.78 |
| 7 | 15.782 | 35.84 | 21 | 27.578 | 8.31 |
| 8 | 16.288 | 81.69 | 22 | 29.478 | 2.46 |
| 9 | 17.314 | 29.70 | 23 | 31.215 | 3.05 |
| 10 | 17.575 | 65.67 | 24 | 31.522 | 2.85 |
| 11 | 17.819 | 100.00 | 25 | 33.184 | 1.67 |
| 12 | 18.204 | 7.25 | 26 | 34.609 | 3.25 |
| 13 | 19.192 | 25.27 | 27 | 35.313 | 1.53 |
| 14 | 19.414 | 9.40 | - | - | - |

### Test Example 1. In Vitro Evaluation of Intrasynaptic and Extrasynaptic Functional Activities in Cells

### 1.1. Experimental materials

| Material name | Manufacturer | Cat. No. |
|---|---|---|
| α4β3δ-HEK-FlpIn-TRex | Beijing Institute of Materia Medica | / |
| α1β2γ2-CHO-TRex | Beijing Institute of Materia Medica | / |
| GABA | Sigma | A2129 |
| DMEM culture medium | Invitrogen | C11995500BT |
| Fetal bovine serum | Shanghai Yeasen | 40130ES76 |
| Blasticidin | Shanghai Yeasen | 60218ES10 |
| Zeocin | Invitrogen | R25001 |
| Hygromycin | Shanghai Yeasen | 60225ES10 |
| Puromycin | Solarbio | P8230 |
| Tetracycline | Sigma | 87128 |
| Polylysine | Sigma | P1149 |
| Trypsin | Gibco | 27250018 |

### 1.2. Experimental method

The α4β3δ-HEK-FlpIn-TRex stable cell strain was cultured in a DMEM + 10% FBS complete culture medium, with 10 µg/mL blasticidin, 100 µg/mL zeocin, 100 µg/mL hygromycin, and 0.2 µg/mL puromycin as selective antibiotics. The α1β2γ2-CHO-TRex stable cell strain was cultured in a DMEM/F12 + 10% FBS complete culture medium, with 10 µg/mL blasticidin, 100 µg/mL zeocin, 300 µg/mL hygromycin, and 1 µg/mL puromycin as selective antibiotics. The day before the patch clamp experiment, cells were seeded onto 12 mm polylysine-coated coverslips and then cultured in 35 mm culture dishes, and tetracycline (1 µg/mL) was added to induce the expression of GABA receptors. The extracellular fluid had the following composition (in mM): 140 NaCl, 3 KCl, 1.5 MgCl₂, 2 CaCl₂, 10 HEPES, and 10 glucose. After thorough mixing, NaOH was added to adjust the pH to 7.4, and sucrose was added to adjust the osmotic pressure to 300-320 mOsm. The fluid was stored at 4 °C. The electrode internal solution had the following composition (in mM): 145 KCl, 1 MgCl₂, 5 EGTA, 10 HEPES, and 5 MgATP. After thorough mixing, KOH was added to adjust the pH to 7.3, and sucrose was added to adjust the osmotic pressure to 290-300 mOsm. The solution was filtered through a 0.22 µm filter before use.

This experiment mainly employed whole-cell patch clamp recording. The membrane potential was clamped at -60 mV, and receptors were activated using GABA at an EC10-20 concentration (~1 µM). The test compound was dissolved in 0.1% DMSO, and perfusion was performed for 30 s before GABA stimulation. Finally, the positive allosteric regulatory effect of the test compound on α1β2γ2 or α4β3δ was determined.

The experimental result for each concentration was expressed as a fold increase in the GABA-induced current caused by the compound. Initially, the minimum concentration at which the compound was just able to enhance the GABA-induced current was determined, and then, the test concentration was gradually increased (3-fold increases) until the activating effect of the compound reached the EC₈₀ effect or higher. At least 5 concentrations are tested to obtain a complete EC₅₀ curve and the EC₅₀ value. All experiments were repeated on no less than 3 cells, and data were processed using GraphPad prism software. Dose-response curves were fitted using the Hill equation, and the halfmaximal activating concentration EC₅₀ and Eₘₐₓ were finally obtained. The results are shown in Table 5.

**Table 5. The functional activity of the compound of formula I on intrasynaptic and extrasynaptic receptors**

| **Compound name** | **Intrasynaptic functional activity (α1β2γ2) (EC₅₀, nM/Eₘₐₓ, %)** | **Extrasynaptic functional activity (α4β3δ) (EC₅₀, nM/Eₘₐₓ, %)** |
|---|---|---|
| Compound of formula I | 145/762 | 67/754 |

The results show that the compound of formula I exhibited relatively good intrasynaptic and extrasynaptic functional activities.

### Test Example 2. In Vitro Liver Microsomal Stability Evaluation

### 2.1. Solution preparation

1) Preparation of a working solution of the test sample: The test sample was diluted with acetonitrile to 100 µM.
2) Preparation of a working solution of liver microsomes: Liver microsomes were diluted with 100 mM phosphate-buffered saline to 0.56 mg/mL.
3) Preparation of a working solution of reduced nicotinamide adenine dinucleotide phosphate (NADPH): A proper amount of NADPH was weighed out and diluted with phosphate-buffered saline to 20 mM, and an equal volume of a 60 mM MgCl₂ solution was added.
4) Preparation of a stop solution: Tolbutamide was diluted with acetonitrile to 20 ng/mL. The resulting solution was used as a stop solution containing an internal standard.

### 2.2. Incubation process

1) Incubation anti-adsorption EP tubes were prepared and labeled with the species, test/reference sample, time points (0, 5, 10, 20, 30, 60 min, Blank60, and NCF60), etc.
2) 2 µL of the test or reference sample working solution (diluted with acetonitrile to 100 µM) and 178 µL of the liver microsome working solution were added to each tube. For the Blank60 tube, 2 µL of acetonitrile was added instead of the test sample. The tubes were pre-incubated in a water bath at 37 °C for about 10 min, with each sample tested in triplicate.
3) After the pre-incubation, 20 µL of the NADPH working solution was added to each tube, except for the 0 min and NCF60 tubes, to start reactions, and 20 µL of phosphate-buffered saline (containing 30 mM MgCl₂) was added to the NCF60 tube. In the incubation system, the final concentration of the test or reference sample was 1 µM, the final concentration of liver microsomes was 0.5 mg/mL, the final concentration of NADPH was 1 mM, and the final concentration of MgCl₂ was 3 mM.
4) For the 0 min sample, 600 µL of the stop solution was added, and the NADPH working solution was then added. For the other samples, after corresponding periods of incubation, 600 µL of the stop solution was added to stop the reaction.
5) After the reaction of each sample was stopped, the reaction mixtures were vortexed for 30 s and then centrifuged at 13,500 rpm for 10 min. The supernatants (100 µL) were transferred to EP tubes, and 100 µL of Milli-Q water was added. After thorough mixing by vortexing, the mixtures were analyzed by LC-MS/MS.
6) Testosterone and dextromethorphan were tested as positive reference samples under the same conditions to assess the stability and reliability of the system.

### 2.3. Data analysis

Parameters of the test sample (its half-life, intrinsic clearance rate in liver microsomes, and hepatic intrinsic clearance rate) were calculated based on the remaining percentage of the test sample. The results are shown in Table 6.

**Table 6. The half-life, intrinsic clearance rate in liver microsomes, and hepatic intrinsic clearance rate of the compound**

| **Compound No.** | **Species** | **Remaining amount at 60 min (%)** | **T_{1/2}(min)** | **CLint(mic) µL/min/mg protein** | **Clint(liver) mL/min/kg** |
|---|---|---|---|---|---|
| Compound of formula I | Rat | 68.3 | 108.3 | 13 | 23 |
| | Human | 98.2 | >186 | <7.50 | <6.75 |

As shown in the table above, the compound of formula I provided by the present disclosure exhibited, for the most part, relatively good stability in liver microsomes of rats and humans, indicating that it has good metabolic stability.

### Test Example 3. Loss of Righting Reflex of SD Rats Injected with Compound of Formula I

### 3.1. Test objective

To determine the righting reflex loss threshold of injection of the compound of formula I into SD rats, so as to evaluate the sedative effect and therapeutic safety window of the compound.

### 3.2. Test process

Male SD rats weighing 180-200 g (test animals) were acclimatized for 7 days after arriving at the animal facility and were randomly divided into groups according to weight one day before the test. Before the test, the animals were fasted for half a day, during which access to water was given. Doses of the compound of formula I were designed with a 0.8-fold gradient: 23.5 mg/kg, 18.8 mg/kg, 15 mg/kg, 12 mg/kg, and 9.6 mg/kg. The loss of the rats' righting reflex was monitored within 4 h of intraperitoneally injecting the compound of formula I. The criterion for determining if the righting reflex was lost was as follows: After injection of the compound of formula I, rats in a supine position that did not right themselves within 30 s were considered positive for loss of the righting reflex. Otherwise, they were considered negative for loss of the righting reflex.

### 3.3. Test results

The test results show that at doses of 23.5 mg/kg, 18.8 mg/kg, 15 mg/kg, 12 mg/kg, and 9.6 mg/kg, the righting reflex loss rates in rats were 80%, 50%, 30%, 0, and 0, respectively. Under the test conditions, the righting reflex loss threshold dose of intraperitoneal injection of the compound of formula I into SD rats was 12 mg/kg, indicating that the compound of formula I has a relatively wide therapeutic safety window.

### Test Example 4. Loss of Righting Reflex of SD Rats Intragastrically Given Compound of Formula I

### 4.1. Test objective

To determine the righting reflex loss threshold of intragastric administration of the compound of formula I to SD rats, so as to evaluate the sedative effect and therapeutic safety window of the compound of formula I.

### 4.2. Test method

Male SD rats weighing 180-200 g (experimental animals) were acclimatized for 7 days after arriving at the animal facility and were randomly divided into groups according to weight one day before the experiment. Before the experiment, the animals were fasted overnight, during which access to water was given. Doses of the compound of formula I were designed with a 0.8-fold gradient: 39 mg/kg, 31.25 mg/kg, 25 mg/kg, 20 mg/kg, and 16 mg/kg, with 10 animals in each group. The loss of the rats' righting reflex was monitored within 4 h of intragastrically administering the compound. The criterion for determining if the righting reflex was lost was as follows: After administration of the test compound, rats in a supine position that did not right themselves within 30 s were considered positive for loss of the righting reflex. Otherwise, they were considered negative for loss of the righting reflex.

### 4.3. Test results

The results show that at doses of 39 mg/kg, 31.25 mg/kg, 25 mg/kg, 20 mg/kg, and 16 mg/kg, the righting reflex loss rates in rats were 100%, 90%, 50%, 0%, and 0%, respectively. Under the experimental conditions, the righting reflex loss threshold dose of intragastric administration of the compound of formula I to SD rats was 20 mg/kg. The righting reflex loss threshold of intragastric administration of the compound of formula I to rats (20 mg/kg) is 5.7 times the effective dose for the antiepileptic convulsion effect (3.5 mg/kg), indicating a relatively wide therapeutic safety window.

### Test Example 5. Pharmacokinetic Study of Compound of Formula I in CD-1 Mice

### 4.1. Test animals and vehicle

Male CD-1 mice weighing about 25 g were randomly divided into groups of 3.
DMSO:30% SBECD (10%:90%)

### 4.2. Test method

The intravenous (IV) dose was 1 mg/kg, and the intragastric (IG) dose was 5 mg/kg. Before administration, the animals were fasted for 12 h and given free access to water. Three hours after administration, the animals were fed together. Blood samples (about 0.15 mL) were collected at 5 min (intravenous),15 min, 30 min, 1 h, 2 h, 4 h, 8 h, 12 h, and 24 h post-dose into heparinized EP tubes and centrifuged at 13,500 rpm for 10 min to separate the plasma. Mouse brain tissue samples were collected after cardiac perfusion at 30 min, 2 h, and 8 h. After pretreatment, the samples were analyzed by LC-MS/MS to determine the concentration of the test compound in the plasma and brain, and pharmacokinetic parameters were calculated.

### 4.3. Test results

**Table 7. Pharmacokinetic parameters of the compound of formula I in CD-1 mice after intravenous administration (1 mg/kg) and intragastric administration (5 mg/kg)**

| Route of administration | Tₘₐₓ | C₀/Cₘₐₓ | AUCₗₐₛₜ | AUC_{inf} | T_{1/2} | MRTₗₐₛₜ | F |
|---|---|---|---|---|---|---|---|
| | hour | ng/mL | h*ng/mL | h*ng/mL | hour | hour | % |
| IV | / | 490 | 3661 | 4316 | 9.0 | 7.9 | / |
| IG | 1 | 1673 | 20016 | 31341 | 16.5 | 9.8 | 109 |

**Table 8. The concentrations of the compound of formula I in the brains of CD-1 mice after intragastric administration (5 mg/kg)**

| | 30 min | 2 h | 8 h |
|---|---|---|---|
| Brain (ng/g) | 847±36 | 1917±978 | 1589±233 |
| Plasma (ng/mL) | 936±119 | 1284±345 | 891±72 |
| Brain-to-plasma ratio | 0.9 | 1.5 | 1.8 |

The data in Table 7 show that both intravenous administration and intragastric administration of the compound of formula I achieved relatively high plasma exposure levels (AUC) and relatively long half-lives in CD-1 mice, and the absolute bioavailability of intragastric administration reached 109%, indicating that the compound of formula I has good *in vivo* metabolic stability and exhibits high levels of *in vivo* absorption. The data in Table 8 show that the compound of formula I exhibited a strong brain penetration ability, which led to high brain exposure levels and relatively high concentrations in the brain.

### Test Example 6. Pharmacokinetic Study of Compound of Formula I in SD Rats

### 5.1. Test animals and vehicle

Male SD rats weighing about 250 g were randomly divided into groups of 3.
DMSO:30% SBECD (10%:90%)

### 5.2. Test method

The intravenous dose was 6.1 µmol/kg, and the intragastric dose was 24.4 µmol/kg. Before administration, the animals were fasted for 12 h and given free access to water. Three hours after administration, the animals were fed together. Blood samples (about 0.3 mL) were collected at 5 min (intravenous), 15 min, 30 min, 1 h, 2 h, 3 h, 4 h, 6 h, 8 h, 12 h, and 24 h post-dose into heparinized EP tubes and centrifuged at 13,500 rpm for 10 min to separate the plasma. Rat brain tissue samples were collected after cardiac perfusion at 30 min, 2 h, and 6 h. After pretreatment, the samples were analyzed by LC-MS/MS to determine the concentration of the test compound in the plasma and brain, and pharmacokinetic parameters were calculated.

### 5.3. Test results

**Table 9. Pharmacokinetic parameters of the compound of formula I in SD rats after intravenous administration (6.1 µmol/kg) and intragastric administration (24.4 µmol/kg)**

| Route of administration | Tₘₐₓ | C₀/Cₘₐₓ | AUCₗₐₛₜ | AUC_{inf} | T_{1/2} | MRTₗₐₛₜ | F |
|---|---|---|---|---|---|---|---|
| | hour | nmol/L | hour*nmol/L | hour*nmol/L | hour | hour | % |
| IV | / | 2112±537 | 9710±2259 | 10050±2377 | 5.17±0.55 | 6.3±0.83 | / |
| IG | 1.67±0.58 | 3277±324 | 32520±5197 | 35123±7031 | 6.11±1.44 | 7.61±0.6 | 84 |

**Table 10. The concentrations of the compound of formula I in the brains of SD rats after intragastric administration (24.4 µmol/kg)**

| | 30 min | 2 h | 6 h |
|---|---|---|---|
| Brain (nmol/kg) | 2565±452 | 4533±720 | 2692±259 |
| Plasma (nmol/L) | 3203±1159 | 3813±670 | 1475±261 |
| Brain-to-plasma ratio | 0.8 | 1.2 | 1.8 |

The data in Table 9 show that both intravenous administration and intragastric administration of the compound of formula I achieved relatively high plasma exposure levels (AUC) and relatively long half-lives in SD rats, and the absolute bioavailability of intragastric administration was 84%, indicating that the compound of formula I has good *in vivo* metabolic stability and exhibits high levels of *in vivo* absorption. The data in Table 10 show that the compound of formula I exhibited a strong brain penetration ability, which led to high brain exposure levels and relatively high concentrations in the brain.

### Test Example 7. Evaluation of Anticonvulsant Effect of Compound of Formula I in SD Rats

### 6.1. Test objective

To establish an SD rat animal model of epileptic convulsions by intraperitoneally injecting pentylenetetrazol (PTZ) (50 mg/kg) into SD rats, and to evaluate the anticonvulsant effect of the compound of formula I.

### 6.2. Test method

Male SD rats weighing 300±10 g (test animals) were acclimatized for 7 days after arriving at the animal facility and were randomly divided into groups according to weight one day before administration. Before the test, the animals were fasted for half a day, during which access to water was given. In the test, the compound of formula I was orally administered to the animals at 3.5 mg/kg in advance. Two hours after oral administration, a PTZ solution was intraperitoneally injected at 50 mg/kg. The rats' epileptic convulsions within 1 h of performing PTZ induction were monitored. The latency period of clonic convulsions and the latency period of tonic-clonic seizures were measured.

### 6.3. Test results

Behavioral raw data are finally expressed as mean ± standard error (Mean ± S.E.M.) and statistically analyzed using One-way ANOVA Dunnett post hoc. *P* < 0.05 indicates the presence of a significant difference, *P* < 0.01 indicates the presence of a very significant difference, and *P* < 0.001 indicates the presence of an extremely significant difference.

The results of the monitoring of epileptic convulsions show that within 1 h of intraperitoneally injecting PTZ at 50 mg/kg to induce epileptic convulsions, the latency period of clonic convulsions in the animals of the treatment group (2764.6±1330.9 s) was significantly greater than the latency period of the solvent control group (114.5±36.9 s) (*P* < 0.001); the latency period of tonic-clonic seizures in the animals of the treatment group (3600±0 s) was significantly greater than the latency period of the solvent control group (115.8±37.1 s) (*P* < 0.001).

## Claims

1. A crystal form A of a compound of formula I, wherein the crystal form A has, in an X-ray powder diffraction pattern using Cu-Kα radiation, characteristic diffraction peaks at the following 2θ angles: 6.752±0.2°, 13.684±0.2°, 15.748±0.2°, and 17.744±0.2°, preferably characteristic diffraction peaks at the following 2θ angles: 6.752±0.2°, 13.684±0.2°, 15.483±0.2°, 15.748±0.2°, and 17.744±0.2°, and more preferably characteristic diffraction peaks at the following 2θ angles: 6.752±0.2°, 9.943±0.2°, 12.794±0.2°, 13.684±0.2°, 14.750±0.2°, 15.483±0.2°, 15.748±0.2°, and 17.744±0.2°; most preferably, the X-ray powder diffraction pattern using Cu-Kα radiation is as shown in FIG. 1-1:

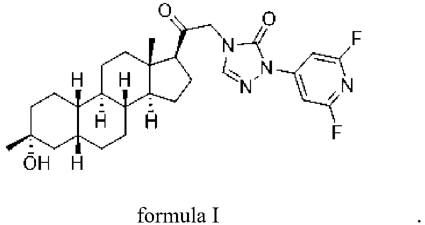

2. A crystal form B of a compound of formula I, wherein the crystal form B has, in an X-ray powder diffraction pattern using Cu-Kα radiation, characteristic diffraction peaks at the following 2θ angles: 15.962±0.2°, 16.497±0.2°, 17.275±0.2°, and 17.757±0.2°, preferably characteristic diffraction peaks at the following 2θ angles: 11.753±0.2°, 15.312±0.2°, 15.962±0.2°, 16.497±0.2°, 17.275±0.2°, 17.757±0.2°, 18.395±0.2°, 19.191±0.2°, 23.754±0.2°, and 24.571±0.2°, and more preferably characteristic diffraction peaks at the following 2θ angles: 11.428±0.2°, 11.753±0.2°, 15.312±0.2°, 15.962±0.2°, 16.497±0.2°, 17.275±0.2°, 17.757±0.2°, 19.191±0.2°, 23.754±0.2°, 24.571±0.2°, and 25.672±0.2°; most preferably, the X-ray powder diffraction pattern using Cu-Kα radiation is as shown in FIG. 2-1.

3. The crystal form B according to claim 2, wherein the crystal form B is a hydrate crystal form.

4. A methanol solvate crystal form C of a compound of formula I, wherein the methanol solvate crystal form C has, in an X-ray powder diffraction pattern using Cu-Kα radiation, characteristic diffraction peaks at the following 2θ angles: 15.574±0.2°, 16.065±0.2°, 17.384±0.2°, and 17.834±0.2°, preferably characteristic diffraction peaks at the following 2θ angles: 11.112±0.2°, 11.631±0.2°, 15.574±0.2°, 16.065±0.2°, 17.384±0.2°, 17.834±0.2°, 19.166±0.2°, 23.507±0.2°, 23.791±0.2°, and 24.810±0.2°; more preferably, the X-ray powder diffraction pattern using Cu-Kα radiation is as shown in FIG. 3-1.

5. The methanol solvate crystal form C of the compound of formula I according to claim 4, wherein the methanol solvate crystal form C of the compound of formula I has a molar ratio of MeOH to the compound of formula I of about 0.8-1.2:1.

6. A methanol solvate crystal form D of a compound of formula I, wherein the methanol solvate crystal form D has, in an X-ray powder diffraction pattern using Cu-Kα radiation, characteristic diffraction peaks at the following 2θ angles: 15.782±0.2°, 16.288±0.2°, 17.575±0.2°, and 17.819±0.2°, preferably characteristic diffraction peaks at the following 2θ angles: 11.731±0.2°, 15.398±0.2°, 15.782±0.2°, 16.288±0.2°, 17.314±0.2°, 17.575±0.2°, 17.819±0.2°, and 19.192±0.2°; more preferably, the X-ray powder diffraction pattern using Cu-Kα radiation is shown in FIG. 4-1.

7. The crystal form D of the compound of formula I according to claim 6, wherein the methanol solvate crystal form D of the compound of formula I has a molar ratio of MeOH to the compound of formula I of about 0.5.

8. A pharmaceutical composition, comprising the crystal form according to any one of claims 1-7 and a pharmaceutically acceptable carrier.

9. Use of the crystal form according to any one of claims 1-7 or the pharmaceutical composition according to claim 8 in the preparation of a GABAA receptor modulator medicament.

10. Use of the crystal form according to any one of claims 1-7 or the pharmaceutical composition according to claim 8 in the preparation of a medicament for preventing and/or controlling central nervous system-related diseases or disorders.

11. The use according to claim 10, wherein the central nervous system diseases or disorders include sleep disorder, depression, mood disorder, manic disorder, dysthymic disorder, bipolar disorder, anxiety disorder, stress, post-traumatic stress disorder (PTSD), compulsive disorder, schizophrenia spectrum disorder, convulsive disorder, memory and/or cognitive disorders, dementia, movement disorder, personality disorder, autism, autism spectrum disorder (ASD), pain, traumatic brain injury (TBI), vascular diseases, substance abuse disorder and/or withdrawal syndrome, and tinnitus.

12. The use according to claim 11, wherein the depression includes minor depression, major depressive disorder, persistent depressive disorder, psychotic depression, postpartum depression, or seasonal affective disorder.

13. The use according to claim 10, wherein the central nervous system-related diseases include insomnia, bipolar I disorder, bipolar II disorder, generalized anxiety disorder (GAD), social anxiety disorder, schizophrenia, schizoaffective disorder, attention disorder, dementia of the Alzheimer type, dementia with Lewy bodies, vascular dementia, Huntington's disease, Parkinson's disease, essential tremor, antisocial personality disorder, obsessive-compulsive personality disorder, autism, monogenetic causes of autism, synaptophathy, Rett syndrome, fragile X syndrome, Angelman syndrome, neuropathic pain, injury-related pain syndrome, acute pain, chronic pain, stroke, ischemia, vascular malformation, and addiction to opiates, cocaine, and/or alcohol.
